(19) Europäisches Patentamt
European Patent Office
Office européen des brevets

(11) Publication number: **0 669 320 A2**

(12) **EUROPEAN PATENT APPLICATION**

(21) Application number: **95102073.4**

(51) Int. Cl.[6]: **C07D 215/18**, A01N 43/42

(22) Date of filing: **15.02.95**

(30) Priority: **28.02.94 JP 52544/94**
**01.08.94 JP 197176/94**

(43) Date of publication of application:
**30.08.95 Bulletin 95/35**

(84) Designated Contracting States:
**BE CH DE DK ES FR GB IT LI NL PT**

(71) Applicant: **NIHON BAYER AGROCHEM K.K.**
**10-8, Takanawa 4-chome**
**Minato-ku**
**Tokyo 108 (JP)**

(72) Inventor: **Kurahashi, Yoshio**
**1-4-14, Kamiyama**
**Oyama-shi,**
**Tochigi (JP)**
Inventor: **Moriya, Koichi, 3300-11, 269-1-7,**
**Yakushiji**
**Oaza,**
**Minamikawachi-machi**
**Kawaachi-gun,**
**Tochigi (JP)**
Inventor: **Sawada, Haruko**
**848-4, Odabayashi**
**Yuki-shi,**
**Ibaraki (JP)**
Inventor: **Sakuma, Haruhiko**
**1-5-7, Ekinancho**
**Oyama-shi,**
**Tochigi (JP)**
Inventor: **Wada, Katsuaki**
**7-11-20, Joto**
**Oyama-shi,**
**Tochigi (JP)**
Inventor: **Watanabe, Ryo**
**1057-1, Inabago**
**Oyama-shi,**
**Tochigi (JP)**
Inventor: **Ito, Asami**
**934-7, Hitotonoya**
**Oyama-shi,**
**Tochigi (JP)**

(74) Representative: **Linkenheil, Dieter et al**
**Bayer AG**
**Patente Konzernzentrale RP**
**D-51368 Leverkusen (DE)**

(54) **Benzoylquinoiline derivatives.**

(57) Novel benzoylquinoline derivatives of the formula

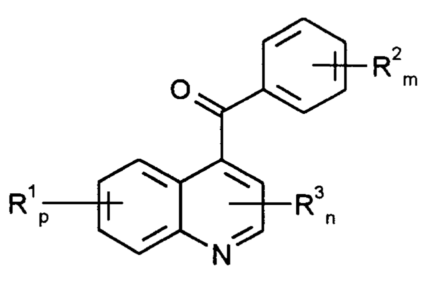

(I)

wherein

$R^1$ represents halogen, $C_{1-4}$ alkyl, $C_{1-4}$ haloalkoxy or $C_{1-4}$ haloalkyl,

p represents an integer of 0 or 1,

EP 0 669 320 A2

$R^2$ represents halogen, nitro, cyano, $C_{1-4}$ alkyl, $C_{1-4}$ alkoxy, $C_{1-4}$ alkylthio, $C_{1-4}$ haloalkyl, $C_{1-4}$ haloalkoxy or phenyl,

and

when m represents 2, then $R^2$ may also represent methylenedioxy,

m represents an integer of 0, 1, 2, 3, 4 or 5,

$R^3$ represents $C_{1-4}$ alkyl or $C_{1-4}$ haloalkyl, and

n represents an integer of 0 or 1,

and acid addition salts and metal salt complexes thereof,

processes for the preparation of the new compounds and their use as fungicides.

The present invention relates to new benzoylquinoline derivatives, to processes for their preparation and to their use as fungicides.

It is already known that certain quinoline derivatives are useful as fungicides (see Japanese Patent Applications Laid-Open Nos. Hei 1-246 263, Hei 3-66 689, Hei 4-187 677, Hei 5-202 o32 and Hei 5-27 122).

There have been found novel benzoylquinoline derivatives of the formula

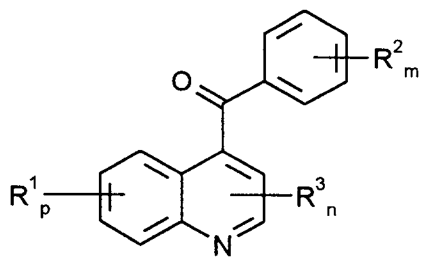

(I)

wherein

- $R^1$ represents halogen, $C_{1-4}$ alkyl, $C_{1-4}$ haloalkoxy or $C_{1-4}$ haloalkyl,
- p represents an integer of 0 or 1,
- $R^2$ represents halogen, nitro, cyano, $C_{1-4}$ alkyl, $C_{1-4}$ alkoxy, $C_{1-4}$ alkylthio, $C_{1-4}$ haloalkyl, $C_{1-4}$ haloalkoxy or phenyl,

and

when m represents 2, then $R^2$ may also represent methylenedioxy,

- m represents an integer of 0, 1, 2, 3, 4 or 5,
- $R^3$ represents $C_{1-4}$ alkyl or $C_{1-4}$ haloalkyl, and
- n represents an integer of 0 or 1,

and acid addition salts and metal salt complexes thereof.

It has furthermore been found that benzoylquinoline derivatives of the formula (I) and acid addition salts and metal salt complexes thereof are obtained,

when

(a) 4-chloroquinoline derivatives of the formula

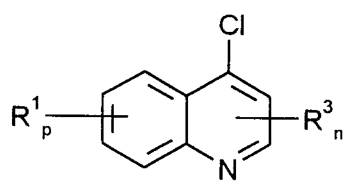

(II)

wherein $R^1$, $R^3$ , n and p have the above mentioned meanings,

are reacted with phenyl acetonyl derivatives of the formula

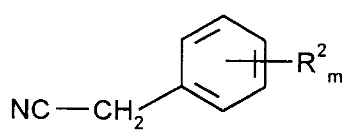

(III)

wherein $R^2$ and m have the above mentioned meanings,

in the presence of oxygen, if appropriate, in the presence of an acid-binder and, if appropriate, in the presence of an inert diluent,

or

(b) quinoline carboxylic acid chloride derivatives of the formula

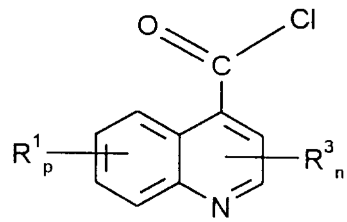

(IV)

wherein

$R^1$, $R^3$, n and p have the above mentioned meanings,

are reacted with compounds of the formula

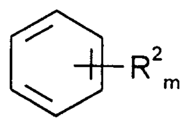

(V)

wherein

$R^2$ and m have the above mentioned meanings,

if appropriate, in the presence of Lewis acids and, if appropriate, in the presence of an inert diluent, and, if appropriate, an acid or a metal salt is then added onto the compounds of the formula (I) thus obtained.

Finally, it has been found that the new benzoylquinoline derivatives of the formula (I) and acid addition salts and metal salt complexes thereof have powerful fungicidal properties.

Surprisingly, the compounds according to the invention have a much better fungicidal activity than the already known compounds which are structurally most similar and have the same type of action.

In the general formula (I) of the benzoylquinoline derivatives according to the present invention and the respective general formulae representing their intermediate compounds employed for the production of the benzoylquinoline derivatives, each of the halogen as well as the halogen parts of the halogenoalkyl and halogenoalkoxy substituents are represented by fluoro, chloro, bromo and iodo, preferably chloro or fluoro.

The $C_{1-4}$ alkyl represents, for example, methyl, ethyl, propyl, or isopropyl, n-(iso-, tert-, or sec)butyl, preferably methyl or ethyl.

The $C_{1-4}$ alkoxy represents, for example, methoxy, ethoxy, propoxy, isopropoxy, or n-(iso-, tert-, or sec)butoxy, preferably methoxy, ethoxy, propoxy or isopropoxy.

The $C_{1-4}$ alkylthio represents, for example, methylthio, ethylthio, propylthio, isopropylthio, or n-(iso-, tert- or sec-)butylthio, preferably methylthio or ethylthio.

The $C_{1-4}$ haloalkyl represents the above-mentioned $C_{1-4}$ alkyl which, however, is substituted, at one to all of the hydrogen atoms, with the same or different halogen atoms and is, for example, trifluoromethyl, difluoromethyl, fluoromethyl, chloromethyl, dichloromethyl, etc., preferably trifluoromethyl, difluoromethyl or chloromethyl.

The $C_{1-4}$ haloalkoxy represents the above mentioned $C_{1-4}$ alkoxy which is, however, substituted, at one to all of the hydrogen atoms, with the same or different halogen atoms and is, for example, trifluoromethoxy, difluoromethoxy, fluoromethoxy, chloromethoxy, dichloromethoxy, etc., preferably trifluoromethoxy or difluoromethoxy.

Among the benzoylquinoline derivatives according to the invention, of the formula (I), preferred compounds are those, in which

$R^1$ represents halogen, $C_{1-3}$ alkyl, $C_{1-3}$ haloalkyl,

p represents an integer of 0 or 1,

$R^2$ represents halogen, nitro, cyano, $C_{1-3}$ alkyl, $C_{1-3}$ alkoxy, $C_{1-3}$ alkylthio, $C_{1-3}$ haloalkyl, $C_{1-3}$ haloalkoxy or phenyl, and

when m represents 2, then

$R^2$ may also represent methylenedioxy,

m represents an integer of 0, 1, 2, 3, 4 or 5,

$R^3$ represents $C_{1-3}$ alkyl or $C_{1-3}$ haloalkyl, and

n represents an integer of 0 or 1.

Particularly preferred benzoylquinoline derivatives of the formula (I) are those, in which

$R^1$ represents halogen, methyl or trifluoromethyl,

p represents the integer 0 or 1,

$R^2$ represents halogen, nitro, cyano, $C_{1-3}$ alkyl, $C_{1-3}$ alkoxy, $C_{1-3}$ alkylthio, $C_{1-3}$-haloalkyl, $C_{1-3}$ haloalkoxy or phenyl, and

when m represents 2, then

$R^2$ may also represent a methylenedioxy group,

m represents an integer of 0, 1, 2, 3, 4 or 5,

$R^3$ represents methyl or trifluoromethyl, and

n represents the integer 0 or 1.

Very particularly preferred benzoylquinoline derivatives of the formula (I) are those, in which

$R^1$ represents halogen, methyl or trifluoromethyl attached to the 6-, 7- or 8-position of the quinoline ring,

p represents the integer 0 or 1,

$R^2$ represents halogen, nitro, cyano, $C_{1-3}$ alkyl, $C_{1-3}$ alkoxy, $C_{1-3}$ alkylthio, $C_{1-3}$ haloalkyl, $C_{1-3}$ haloalkoxy or phenyl,

and when m represents 2, then

$R^2$ may also represent a methylenedioxy group,

m represents an integer of 0, 1, 2, 3, 4 or 5,

$R^3$ represents methyl or trifluoromethyl, and

n represents the integer 0 or 1.

Addition products of acids and those benzoylquinoline derivatives of the formula (I), in which $R^1$, $R^2$, $R^3$, m, n and p have the meanings which have already been mentioned as preferred for these radicals and these indices, are also preferred compounds according to the invention.

The acids which can be added on include, preferably, hydrogen halide acids, such as, for example, hydrochloric acid and hydrobromic acid, in particular hydrochloric acid, and, furthermore, phosphoric acid, nitric acid, sulphuric acid, mono- and bifunctional carboxylic acids and hydroxycarboxylic acids, such as, for example, acetic acid, maleic acid, succinic acid, fumaric acid, tartaric acid, citric acid, salicylic acid, sorbic acid and lactic acid, and sulphonic acids, such as, for example p-toluenesulphonic acid, 1,5-naphthalenedisulphonic acid or camphorsulphonic acid, as well as saccharin and thiosaccharin.

Addition products of salts of metals of main groups II to IV and of sub-groups I and II and IV or VIII of the periodic table of the elements and those benzoylquinoline derivatives of the formula (I), in which $R^1$, $R^2$, $R^3$, m, n and p have the meanings which have already been mentioned as preferred for these radicals and these indices, are also preferred compounds according to the invention.

Salts of copper, zinc, manganese, magnesium, tin, iron and nickel are particularly preferred here. Possible anions of these salts are those which are derived from those acids which lead to physiologically acceptable addition products, Particularly preferred acids of this type are, in this connection, the hydrogen halide acids, such as, for example, hydrochloric acid and hydrobromic acid, and, furthermore, phosphoric acid, nitric acid and sulphuric acid.

The substances listed in the following Table 1 may be mentioned as examples of benzoylquinoline derivatives of the formula (I).

**Table 1**

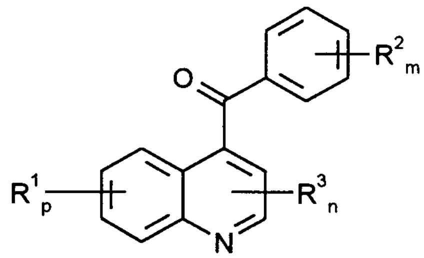

(I)

| $R^1_p$ | $R^2_m$ | $R^3_n$ |
|---|---|---|
| 7-Cl | 2'-Cl | - |
| 7-Cl | 3'-Cl | - |
| 7-Cl | 4-Cl | - |
| 7-Cl | 2'-F | - |
| 7-Cl | 3'-F | - |
| 7-Cl | 4'-F | - |
| 7-Cl | 4'-$OCH_3$ | - |
| 7-Cl | 4'-$CH_3$ | - |
| 7-Cl | - | - |
| 7-Cl | 2',4'-$Cl_2$ | - |
| 7-Cl | 3',4'-$Cl_2$ | - |
| 6-Cl | 4'-Cl | - |

Table 1 (continued)

| $R^1{}_p$ | $R^2{}_m$ | $R^3{}_n$ |
|---|---|---|
| 8-Cl | 4'-Cl | - |
| - | 4'-Cl | - |
| - | 4'-F | - |
| - | 4'-F | 2-$CH_3$ |
| 7-$CH_3$ | 4'-Cl | - |
| 7-$CF_3$ | 4'-Cl | - |
| 7-Cl | 3',4'-$F_2$ | - |
| 7-Cl | 4'-Cl | 2-$CH_3$ |
| 7-Cl | 4'-Cl | 3-$CH_3$ |
| 7-Cl | 2-$OCH_3$ | - |
| 7-Cl | 3-$OCH_3$ | - |
| 7-Cl | 2'-$CH_3$ | - |
| 7-Cl | 3'-$CH_3$ | - |
| 7-F | 4'-$CH_3$ | - |
| 7-F | 4'-Br | - |
| 7-Cl | 4'-$CF_3$ | - |
| 7-Cl | 3'-$CF_3$ | - |
| 7-Cl | 3'-$C_6H_5$ | - |
| 7-Cl | 4'-$NO_2$ | - |
| 7-Cl | 2'-$NO_2$ | - |
| 7-Cl | 2'-CN | - |
| 7-Cl | 4'-CN | - |

Table 1 (continued)

| $R^1{}_p$ | $R^2{}_m$ | $R^3{}_n$ |
|---|---|---|
| 7-Cl | 2'-,6'-$F_2$ | - |
| 7-Cl | 2'-Cl, 6'-F | - |
| 7-Cl | 2', 6'-$Cl_2$ | - |
| 7-Cl | 2',4'-$F_2$ | - |
| 7-Cl | 2'-,5'-$F_2$ | - |
| 7-Cl | 3',4'-$F_2$ | - |
| 7-Cl | 2',4'-$Cl_2$ | - |
| 7-Cl | 3',4'-$Cl_2$ | - |
| 7-Cl | 3',5'-$(CF_3)_2$ | - |
| 7-Cl | 3',4'-$(OCH_3)_2$ | - |
| 7-Cl | 3'-$OCH_2O$-4' | - |
| 7-Cl | 3',4',5'-$(OCH_3)_3$ | - |
| 7-Cl | 2',3',4',5',6'-$F_5$ | - |
| 7-Cl | 4'-$OCF_3$ | - |
| 7-Cl | 4'-$OCHF_2$ | - |
| 7-$CF_3$ | 2'-F | - |
| 7-$CF_3$ | 3'-F | - |
| 7-Cl | 2'-$SCH_3$ | - |
| 7-Cl | 3'-$SCH_3$ | - |
| 7-Cl | 4'-$SCH_3$ | - |
| 7-Cl | 2'-$OCH_2CH_3$ | - |
| 7-Cl | 3'-$OCH_2CH_3$ | - |
| 7-Cl | 4'-$OCH_2CH_3$ | - |

Table 1 (continued)

| $R^1_p$ | $R^2_m$ | $R^3_n$ |
|---|---|---|
| 7-Cl | 4'-$OCH_2CH_2CH_3$ | - |
| 7-Cl | 4'-$OCH(CH_3)_2$ | - |
| 7-Cl | 4'-Cl | 2-$CF_3$ |
| 7-Cl | 4'-F | 2-$CF_3$ |

When in the process (a), for example, 4,7-dichloroquinoline and 4-chlorobenzylcyanide are used as starting materials, the course of the reaction can be represented by the following equation:

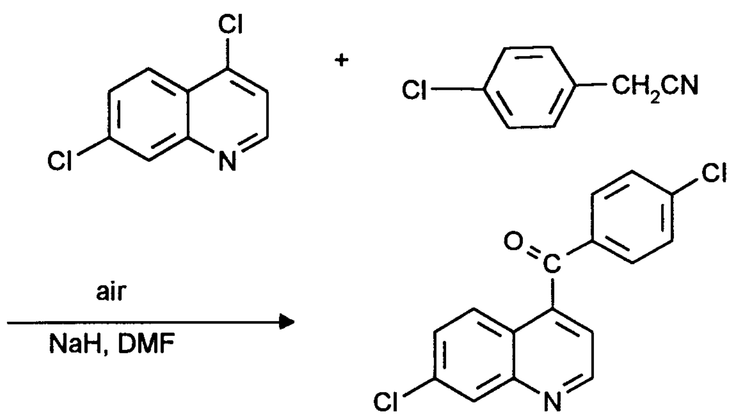


When in the process (b), for example, 7-chloroquinoline-4-carboxylic acid chloride and toluene are used as starting materials, the course of the reaction can be represented by the following equation:

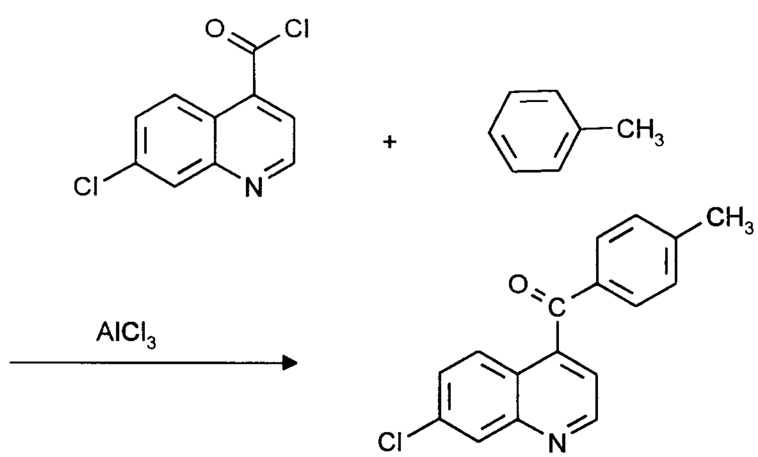


Formula (II) provides a general definition of the 4-chloroquinoline derivatives required as starting materials in process (a) according to the invention. In this formula, $R^1$, $R^3$, n and p preferably have those meanings, which have already been mentioned as preferred for these radicals and these indices in connection with the description of the substances of the formula (I) according to the invention.

The 4-chloroquinoline derivatives represented by the formula (I) are well known in the field of organic chemicals. The 4,7-dichloroquinoline is a compound, which is commercially available. Other 4-chloroquinoline derivatives can be synthesized according to the processes disclosed in Organic Synthesis, pp 272-275, 1955 and Tetrahedron, Vol. 41, pp 3033-3036, 1985.

As specific examples of 4-chloroquinoline derivatives of the formula (II), the following compounds may be mentioned:

4,6-dichloroquinoline, 4,7-dichloroquinoline, 4,8-dichloroquinoline, 4-chloro-7-trifluoromethylquinoline, 4-chloroquinoline, 4-chloro-2-methylquinoline, and 4-chloro-3-methylquinoline.

Formula (III) provides a general definition of the phenyl acetonyl derivatives also required as starting materials in process (a) according to the invention. In this formula, $R^2$ and m preferably have those meanings, which have already been mentioned as preferred for this radical and this index in connection with the description of the substances of the formula (I) according to the invention.

The phenyl acetonyl derivatives of the formula (III) are well known in the field of organic chemicals.

As the examples of the compounds represented by the formula (III), the following compounds may be mentioned:

3-chlorophenylacetonitrile, 3-methoxyphenylacetonitrile, 4-chlorophenylacetonitrile, 4-bromophenylacetonitrile, 2-fluorophenylacetonitrile, 3-phenylphenylacetonitrile, 4-methylphenylacetonitrile, 4-nitrophenylacetonitrile, 3-fluorophenylacetonitrile, 2-nitrophenylacetonitrile, 2-chlorophenylacetonitrile, 2-cyanophenylacetonitrile, 4-fluorophenylacetonitrile, 4-cyanophenylacetonitrile, phenylacetonitrile, 2,6-difluorophenylacetonitrile, 4-methoxyphenylacetonitrile, 2,6-dichlorophenylacetonitrile, 2-methylphenylacetonitrile, 2,4-difluorophenylacetonitrile, 3-methylphenylacetonitrile, 2,5-difluorophenylacetonitrile, 2-methoxyphenylacetonitrile, 2,4-dichlorophenylacetonitrile, 3,4-dichlorophenylacetonitrile, 3,5-bistrifluoromethylphenylacetonitrile, 3,4-difluorophenylacetonitrile, 2-trifluoromethylphenylacetonitrile, 3-trifluoromethylphenylacetonitrile, 4-trifluoromethylphenylacetonitrile, 2-chloro-6-fluorophenylacetonitrile, 3,4-dimethoxyphenylacetonitrile, 3,4,5-trimethoxyphenylacetonitrile, 2,3,4,5,6-pentafluorophenylacetonitrile, 4-trifluoromethoxyphenylacetonitrile, 4-difluoromethoxyphenylacetonitrile, 3,4-(methylenedioxy)-phenylacetonitrile.

Formula (IV) provides a general definition of the quinoline carboxylic acid chloride derivatives required as starting materials in process (b) according to the invention. In this formula, $R^1$, $R^3$, n and p preferably have those meanings, which have already been mentioned as preferred for these radicals and these indices in connection with the description of the substances of the formula (I) according to the invention. 7-chloroquinoline-4-carboxylic acid chloride may be mentioned as an example of a compound of the formula (IV).

The quinoline carboxylic acid chloride derivatives of the formula (IV) are known or can be prepared according to known processes.

Formula (V) provides a general definition of the compounds also required as starting materials in process (b) according to the invention. In this formula, $R^2$ and m preferably have those meanings, which have already been mentioned as preferred for this radical and this index in connection with the description of the substances of the formula (I) according to the invention.

The following compounds may be mentioned as examples of compounds of the formula (V):

Toluene, chlorobenzene, anisole, fluorobenzene, bromobenzene, ethoxybenzene, thioanisole and so on.

The compounds of the formula (V) are well known in the field of organic chemistry.

Process (a) according to the invention is carried out in the presence of oxygen. Said oxygen can be introduced in pure form or in the form of air.

In carrying out the above-mentioned process (a), it is possible to use any inert organic solvents as the suitable diluents. Examples of the diluents are: aliphatic, cycloaliphatic and aromatic hydrocarbons which may be chlorinated hydrocarbons, such as pentane, hexane, cyclohexane, petroleum ether, ligroin, benzene, toluene, xylene, dichloromethane, chlorobenzene, dichlorobenzene, and the like; ethers such as ethyl ether, methyl ethyl ether, isopropyl ether, butyl ether, dioxane, dimethoxyethane (DME), tetrahydrofuran (THF), diethylene glycol dimethylether (DGM), and the like; ketones such as acetone, methyl ethyl ketone (MEK), methyl isopropyl ketone, methyl isobutyl ketone (MIBK), etc.; nitriles such as acetonitrile, propionitrile, acrylonitrile and the like; alcohols such as methanol, ethanol, isopropanol, butanol, ethylene glycol, and the like; esters such as, ethyl acetate, amyl acetate, etc.; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like; and bases such as pyridine, etc.

In carrying out the above-mentioned process (a), use may be made of an acid-binding agent selected from the group consisting of all customary inorganic and organic bases.

As examples of appropriate inorganic bases, there may be mentioned hydroxides, carbonates, and bicarbonates of alkali metalsand alkaline earth metals such as, for example, sodium hydrogen carbonate, potassium hydrogen carbonate, sodium carbonate, potassium carbonate, lithium hydroxide, sodium hydroxide, potassium hydroxide, and calcium hydroxide. Also may be mentioned amides of alkali metals such as lithium amide, sodium amide, potassium amide and the like, for example.

As examples of suitable organic bases there may be mentioned tertiary amines, dialkylamino anilines and pyridines such as, for example, triethylamine, 1,1,4,4-tetramethylethylene diamine (TMEDA), N,N-dimethylaniline, N,N-diethylaniline, pyridine, 4-dimethylamino pyridine (DMAP), 1,4-diazabicyclo-[2,2,2]-octane (DABCO), 1,8-diazabicyclo[5,4,0]undec-7-ene (DBU) and so on.

As further examples of suitable organic bases, there may be mentioned organic lithium compounds, such as methyl lithium, n-butyl lithium, sec-butyl lithium, tert-butyl lithium, phenyl lithium, dimethyl copper lithium, lithium diisopropylamide, lithium cyclohexylisopropylamide, lithium dicyclohexylamide, n-butyl lithium DABCO, n-butyl lithium DBU, n-butyl lithium TMEDA and so on.

In carrying out the above-mentioned process (a), the reaction temperature can be varied within a substantially wide range. For instance, the reaction may be effected at a temperature between about -70 °C and about +200 °C, preferably at a temperature of from about 20 °C to about 120 °C. It is preferred to carry out the reaction under normal pressure, although the use of a higher or lower pressure is also allowable.

In carrying out the reaction of the above-mentioined process (a), use is made, for example, of from 1 to 10 mol amount of phenylaceto nitriles represented by the above-mentioned general formula (III) in a diluent such as DMF for example, per one mol of quinoline derivative represented by the above-mentioned general formula (II) in the presence of oxygen and a base such as sodium hydride, for example, in the amount from 1.0 to 10 mol amount to obtain the aimed compound.

As suitable diluents to be used in carrying out the above-mentioned process (b), there may be mentioned any inert organic solvents.

Examples of the diluents are: aliphatic, cycloaliphatic and aromatic hydrocarbons which may be chlorinated hydrocarbons, such as pentane, hexane, cyclohexane, petroleum ether, ligroin, dichloromethane, chloroform, carbon tetrachloride, 1,2-dichloroethane, chlorobenzene, dichlorobenzene, and the like; carbon disulfide, nitromethane, nitrobenzene and the like, and others.

The above-mentioned process (b) may be carried out in the presence of Lewis acids, and such Lewis acids may be exemplified by anhydrous aluminum chloride, antimony pentachloride, anhydrous ferric chloride, boron trifluoride, ferrous chloride, titanium tetrachloride, stannic chloride, bismuth trichloride, zinc chloride, mercuric chloride, sulfuric acid, hydrogen fluoride and polyphosphoric acid.

In carrying out the above-mentioned process (b), the reaction temperature can be varied within a substantially wide range. For instance, the reaction may be effected at a temperature between about -70 °C and about 150 °C, preferably at a temperature of from about 20 °C to about 120 °C. It is preferred to carry out the reaction under normal pressure, although the use of a higher or lower pressure is also allowable.

The process (b) can be carried out, for example, by employing a 1 to 100 mol amount of a compound represented by the general formula (V) in a diluent such as nitro methane for example, per 1 mol of a compound represented by the general formula (IV) in the presence of from 1 to 10 mol amount of Lewis acid such as anhydrous aluminum chloride for example, to obtain the desired compound.

The benzoylquinoline derivatives of the formula (I) obtainable by processes according to the invention can be converted into acid addition salts or metal salt complexes.

Those acids which have already been mentioned as preferred acids in connection with the description of the acid addition salts according to the invention can preferably be used to prepare acid addition salts of the compounds of the formula (I).

The acid addition salts of the compounds of the formula (I) can be obtained in a simple manner by customary salt formation methods, for example by dissolving a compound of the formula (I) in a suitable inert solvent and adding the acid, for example hydrochloric acid, and can be isolated in a known manner, for example by filtration, and if appropriate purified by washing with an inert organic solvent.

Those salts of metals which have already been mentioned as preferred metal salts in connection with the description of the metal salt complexes according to the invention can preferably be used to prepare metal salt complexes of the compounds of the formula (I).

The metal salt complexes of the compounds of the formula (I) can be obtained in a simple manner by customary processes, thus, for example, by dissolving the metal salt in alcohol, for example ethanol, and adding the solution to compounds of the formula (I). The metal salt complexes can be isolated in a known manner, for example by filtration, and if appropriate purification by recrystallization.

The active compounds according to the present invention exhibit a powerful fungicidal action, so that the compounds can be used for combating undesirable phytopathogenic fungi.

In general, the active compounds according to the present invention can be used in plant protection for combating Plasmodiophoromycetes, Oomycetes, Chytridiomycetes, Zygomycetes, Ascomycetes, Basidiomycetes and Deuteromycetes. Specifically, the active compounds according to the present invention exhibit marked activity on serious diseases of rice plants, viz., Pellicularia sasakii, and diseases for various plants for yielding farm products, viz., Pellicularia filam entosa, as well as other diseases such as Corticium centrifugum (various plants), Pyricularia oryzae, Xanthomonas oryzae (rice plants), Erwinia aroideae (chinese cabbage), Xanthomonas citri (citrus fruits), Cochliobolus miyabeanus (rice plants), Mycosphaerella musicola (banana tree), Botrytis cinerea (strawberry plants and various other farm product-yielding plants), Plasmopara viticola (grape plants), Glomerella cingulata (grapes, apples and pears), Sclerotinia sclerotiorum (various kinds of vegetables), Colletotrichum lagenarium (melons and cucumbers), Diaporthe citri (citrus fruits), leaf-spot causing fungi such as Alternaria mali, Alternaria solani (potatoes), Alternaria kikuchiana (pears), Venturia inaequalis (apples and others), Venturia pirina (pears) and so on. The active compounds according to the present invention particularly exhibit an excellent fungicidal activity on Erysiphe graminis f. sp. tritici, Erysiphe graminis f. sp. hordei, Sphaerotheca fuliginea, Podosphaera leucotricha, Uncinula necator. Furthermore, the present active compounds can be used as bactericides for combating phytotoxic bacteria such as those of Pseudomonadaceae, Rhyzobiaceae, Enterobacteriaceae, Corybacteriaceae and Streptomycetaceae.

The good toleration, by plants, of the active compounds, at the concentrations required for combating plant diseases, permits treatment of above-ground parts of plants, of vegetative propagation stock and seeds, and of the soil.

The active compounds can be converted into the customary formulations, such as solutions, emulsions, wettable powders, suspensions, powders, foams, pastes, granules, tablets, aerosols, natural and synthetic materials impregnated with active compound, very fine capsules in polymeric substances, coating compositions for use on seed, and formulations used with burning equipment, such as fumigating cartidges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chloro-benzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methylisobutyl ketone or cyclohexanone, or strongly polar solvents, such as dimethylformamide and dimethylsulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, maize cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin hydrolysis products.

Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulations.

It is possible to use colorants such as inorganic pigments, for example, iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 percent by weight of active compound, preferably from 0.5 to 90 per cent by weight.

The active compounds according to the invention can be present in the formulations or in the various use forms as a mixture with other known active compounds, such as fungicides, bactericides, insecticides, acaricides, nematicides, herbicides, bird repellents, growth factors, plant nutrients and agents for improving soil structure.

The active compounds can be used as such or in the form of their formulations or the use forms prepared therefrom by further dilution, such as ready-to-use solutions, emulsions, suspensions, powders, pastes and granules. They are used in the customary manner, for example by watering, immersion, spraying, atomizing, misting, vaporizing, injecting, forming a slurry, brushing on, dusting, scattering, dry dressing, moist dressing, wet dressing, slurry dressing or encrusting.

In the treatment of parts of plants, the active compound concentrations in the use forms can be varied within a substantial range. They are, in general, from 1 to 0.0001% by weight, preferably from 0.5 and 0.001%.

For the treatment of seed, amounts of active compound of 0.001 to 50 g, especially 0.01 to 10 g, are generally employed per kilogram of seed.

For the treatment of soil, active compound concentrations, at the point of action, of 0.00001 to 0.1% by weight, especially of 0.0001 to 0.02%, are generally employed.

The preparation and use of the active compounds according to the invention can be seen from the following examples.

**Example 1**

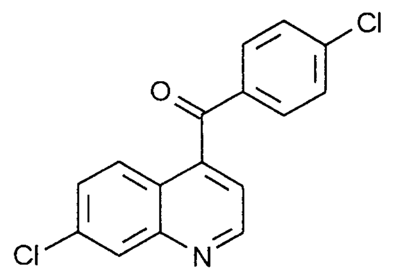

(I-1)

To DMF (50 ml) there were added 4-chlorobenzylcyanide (1.8 g) and sodium hydride (0.8 g), followed by a 30 minute-stirring at room temperature and then 4,7-dichloroquinoline (2.0 g) was added thereto. The resulting mixture was heated to a temperature between 110 to 120 °C for five hours, followed by cooling, addition of water (150 ml) thereto and extraction with ether (200 ml x 3) in that order. The resulting ether layers were put together and dried over anhydrous magnesium sulfate, followed by concentration under reduced pressure and distillation. The resulting reaction product was purified through silica gel column chromatography (n-hexane : ethyl acetate = 9 : 1 v/v) to obtain 7-chloro-4-(4'-chlorobenzoyl)-quinoline (1.6 g).

m.p. 109-109.5 °C

**Example 2**

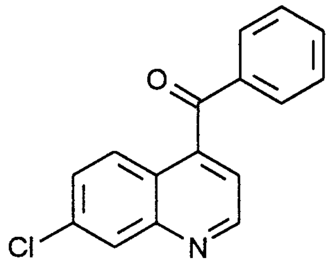

(I-2)

The acid chloride of 7-chloroquinoline-4-carboxylic acid (2.2 g) was dissolved in benzene (100 ml), followed by addition of anhydrous aluminum chloride (3.2 g) thereto. The mixture was then heated for eight hours under refluxing. After the reaction had been completed, the benzene was removed under reduced pressure distillation, followed by addition of 3N hydrochloric acid (50 ml) and separating the crystals formed. The thus obtained crystals were put together under filtration and recrystallized with ethanol to obtain 7-chloro-4-benzoylquinoline (1.2 g).

m.p. 99.5 -102.5 °C

The compounds, which were synthesized by the processes similar to those employed in the foregoing Examples 1 and 2, are shown in the following Table 2:

**Table 2**

| Compound No. | $R^1_p$ | $R^2_m$ | $R^3_n$ | Physical constant |
|---|---|---|---|---|
| (I-3) | 7-Cl | 2'-Cl | - | m.p. 88.5-95°C |
| (I-4) | 7-Cl | 3'-Cl | - | m.p. 97.5-101°C |
| (I-5) | 7-Cl | 2'-F | - | $n_D^{20}$ = 1.6377 |
| (I-6) | 7-Cl | 3'-F | - | $n_D^{20}$ = 1.6424 |
| (I-7) | 7-Cl | 4'-F | - | m.p. 73.5-74.5°C |
| (I-8) | 7-Cl | 4'-$OCH_3$ | - | m.p. 85-86°C |
| (I-9) | 7-Cl | 4'-$CH_3$ | - | $n_D^{20}$ = 1.6515 |
| (I-10) | 7-Cl | 2',4'-$Cl_2$ | - | m.p. 112-115.5°C |
| (I-11) | 7-Cl | 3',4'-$Cl_2$ | - | $n_D^{20}$ = 1.6458 |
| (I-12) | 6-Cl | 4'-Cl | - | m.p. 74-75.5°C |
| (I-13) | 8-Cl | 4'-Cl | - | m.p. 79-82.5°C |
| (I-14) | - | 4'-Cl | - | $n_D^{20}$ = 1.6471 |
| (I-15) | - | 4'-F | 2-$CH_3$ | m.p. 109-116°C |
| (I-16) | - | 4'-F | - | $n_D^{20}$ = 1.6319 |
| (I-17) | 7-$CF_3$ | 4'-Cl | - | $n_D^{20}$ = 1.5828 |
| (I-18) | 7-Cl | 2'-$OCH_3$ | - | m.p. 114.5-115.5°C |
| (I-19) | 7-Cl | 3'-$CF_3$ | - | $n_D^{20}$ = 1.6044 |
| (I-20) | 7-Cl | 3'-$OCH_3$ | - | m.p. 85-92°C |
| (I-21) | 7-Cl | 3',4'-$F_2$ | - | m.p. 91.5-95.0°C |

| Compound No. | $R^1_p$ | $R^2_m$ | $R^3_n$ | Physical constant |
|---|---|---|---|---|
| (I-22) | 7-Cl | 4'-Cl | - | $n_D^{20}$ = 1.6334 |
| (I-23) | 7-F | 4'-F | - | $n_D^{20}$ = 1.5915 |
| (I-24) | 7-F | 3'-F | - | $n_D^{20}$ = 1.6115 |
| (I-25) | 7-Cl | 4'-$NO_2$ | - | m.p. 148.5-154.5°C |

**Example 3**

Application Test on Powdery mildew (Erysiphe graminis f.sp. hordei).

Formulation of active compounds:

The respective active compounds: 30 to 40 parts by weight
Carrier: 55 to 65 parts by weight of diatomaceous earth/kaolin mixture (1:5 mixture)
Emulsifier: 5 parts by weight of polyoxyethylene alkylphenyl ether
To prepare suitable formulations, the stated amount of each of the respective active compounds, the stated amount of the carrier and the stated amount of the emulsifier were ground and mixed to form a wettable powder formulation and, thereafter, a predetermined amount of the wettable powder formulation was diluted with water.

**Test Procedure:**

Seeds of barley (Haruna Nijo variety) were sown in a plurality of pots each having a diameter of 7 cm and made from polyethylene at a rate of 10 seeds per pot and grown at room temperature in the range of from 15 to 25 °C.

After the seeds had grown to small seedlings each having two leaves, they were subjected to spray application at a dosage of 25 ml per three pots with each of the above-mentioned formulations having the predetermined concentration of the active compound. One day after the spray application, the seedlings were subjected to fungi inoculation such that conidia formed on the diseased spots, which had been caused on test plants by previous infection with barley powdery mildew, were dusted onto the above-treated leaves of the seedlings, followed by keeping them at room temperature in the range of from 15 to 20 °C. Seven days after the inoculation, the infection rate per pot was determined according to the following assessment scale to obtain the control values on the basis of three pots on average.

| Infection rate | Area wherein the diseased spots occurred (in %) |
|---|---|
| 0 | 0 |
| 0.5 | less than 2 |
| 1 | 2 - less than 5 |
| 2 | 5 - less than 20 |
| 3 | 10 - less than 20 |
| 4 | 20 - less than 40 |
| 5 | 40 - 100 |

The percent control value was calculated by employing the following equation:

$$\text{Control value (\%)} = \frac{A - B}{A} \times 100$$

wherein
A represents the infection rate in the untreated area;
and
B represents the infection rate in the treated area.

The results of the test:

The compounds (I-1) to (I-8) exhibited a 100% control value each at a dosage of 500 ppm of the active compounds with no phytotoxicity being observed.

**Claims**

**1.** Benzoylquinoline derivatives of the formula

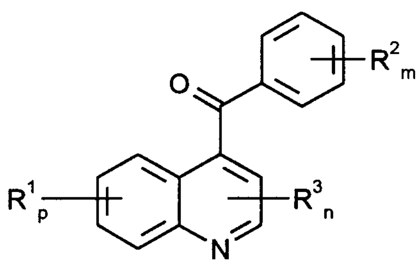

(I)

wherein
$R^1$ represents halogen, $C_{1-4}$ alkyl, $C_{1-4}$ haloalkoxy or $C_{1-4}$ haloalkyl,
p represents an integer of 0 or 1,
$R^2$ represents halogen, nitro, cyano, $C_{1-4}$ alkyl, $C_{1-4}$ alkoxy, $C_{1-4}$ alkylthio, $C_{1-4}$ haloalkyl, $C_{1-4}$ haloalkoxy or phenyl,
and
when m represents 2, then $R^2$ may also represent methylenedioxy,
m represents an integer of 0, 1, 2, 3, 4 or 5,
$R^3$ represents $C_{1-4}$ alkyl or $C_{1-4}$ haloalkyl, and
n represents an integer of 0 or 1,
and acid addition salts and metal salt complexes thereof.

**2.** Compounds of the formula (I) according to claim 1, in which
$R^1$ represents halogen, $C_{1-3}$ alkyl, $C_{1-3}$ haloalkyl,
p represents an integer of 0 or 1,
$R^2$ represents halogen, nitro, cyano, $C_{1-3}$ alkyl, $C_{1-3}$ alkoxy, $C_{1-3}$ alkylthio, $C_{1-3}$ haloalkyl, $C_{1-3}$ haloalkoxy or phenyl, and
when m represents 2, then
$R^2$ may also represent methylendioxy,
m represents an integer of 0, 1, 2, 3, 4 or 5,
$R^3$ represents $C_{1-3}$ alkyl or $C_{1-3}$ haloalkyl, and
n represents an integer of 0 or 1.

**3.** Compounds of the formula (I) according to claim 1, in which
$R^1$ represents halogen, methyl or trifluoromethyl,
p represents the integer 0 or 1,
$R^2$ represents halogen, nitro, cyano, $C_{1-3}$ alkyl, $C_{1-3}$ alkoxy, $C_{1-3}$ alkylthio, $C_{1-3}$ haloalkyl, $C_{1-3}$ haloalkoxy or phenyl,
and when m represents 2, then
$R^2$ may also represent a methylenedioxy group,
m represents an integer of 0, 1, 2, 3, 4 or 5,
$R^3$ represents methyl or trifluoromethyl, and
n represents the integer 0 or 1.

**4.** Compounds of the formula (I) according to claim 1, in which
$R^1$ represents halogen, methyl or trifluoromethyl attached to the 6-, 7 or 8-position of the quinoline ring,
p represents the integer 0 or 1,
$R^2$ represents halogen, nitro, cyano, $C_{1-3}$ alkyl, $C_{1-3}$ alkoxy, $C_{1-3}$ alkylthio, $C_{1-3}$ haloalkyl, $C_{1-3}$ haloalkoxy or phenyl,
and when m represents 2, then
$R^2$ may also represent a methylenedioxy group,
m represents an integer of 0, 1, 2, 3, 4 or 5,
$R^3$ represents methyl or trifluoromethyl, and
n represents the integer 0 or 1.

**5.** Process for the preparation of benzoylquinoline derivatives of the formula

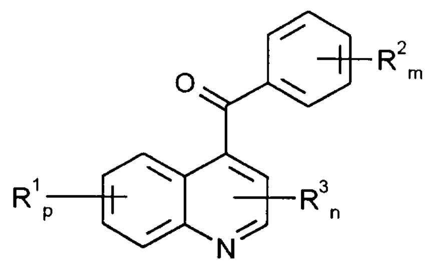

(I)

wherein
$R^1$ represents halogen, $C_{1-4}$ alkyl, $C_{1-4}$ haloalkoxy or $C_{1-4}$ haloalkyl,
p represents an integer of 0 or 1,
$R^2$ represents halogen, nitro, cyano, $C_{1-4}$ alkyl, $C_{1-4}$ alkoxy, $C_{1-4}$ alkylthio, $C_{1-4}$ haloalkyl, $C_{1-4}$ haloalkoxy or phenyl,
and
when m represents 2, then $R^2$ may also represent methylenedioxy,
m represents an integer of 0, 1, 2, 3, 4 or 5,
$R^3$ represents $C_{1-4}$ alkyl or $C_{1-4}$ haloalkyl, and
n represents an integer of 0 or 1,
and acid addition salts and metal salt complexes thereof,
characterized in that
(a) 4-chloroquinoline derivatives of the formula

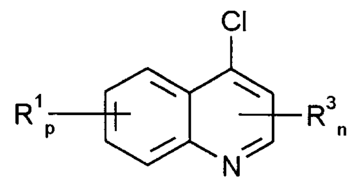

(II)

wherein $R^1$, $R^3$ , n and p have the above mentioned meanings, are reacted with phenyl acetonyl derivatives of the formula

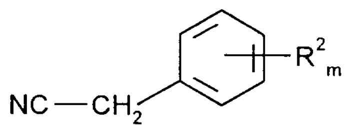

(III)

wherein $R^2$ and m have the above mentioned meanings,
in the presence of oxygen, if appropriate, in the presence of an acid-binder and, if appropriate, in the presence of an inert diluent,
or
(b) quinoline carboxylic acid chloride derivatives of the formula

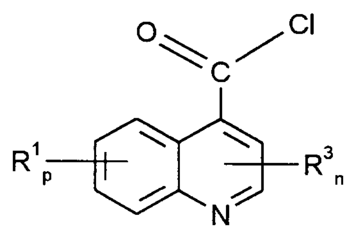

(IV)

wherein
$R^1$, $R^3$, n and p have the above mentioned meanings,
are reacted with compounds of the formula

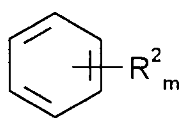

(V)

wherein
$R^2$ and m have the above mentioned meanings,
if appropriate, in the presence of Lewis acids and, if appropriate, in the presence of an inert diluent, and, if appropriate, an acid or a metal salt is then added onto the compounds of the formula (I) thus obtained.

6. Fungicidal compositions, characterized in that they contain at least one benzoylquinoline derivative of the formula (I) according to claim 1 or an acid addition salt or metal salt complex of a benzoylquinoline derivative of the formula (I).

7. Process for combating fungi, characterized in that benzoylquinoline derivatives of the formula (I) according to claim 1 or acid addition salts or metal salt complexes thereof are applied to the fungi and/or to their habitat.

8. Use of benzoylquinoline derivatives of the formula (I) according to claim 1 or acid addition salts of metal salt complexes thereof for combating fungi.

9. Process for the preparation of fungicidal compositions, characterized in that benzoylquinoline derivatives of the formula (I) according to claim 1 or acid addition salts or metal salt complexes thereof are mixed with extenders and/or surface active agents.